# EUROPEAN PATENT SPECIFICATION

(11) **EP 0 496 410 B1**
(45) Date of publication and mention of the grant of the patent: **16.10.1996**
(21) Application number: 92101133.4
(22) Date of filing: 24.01.1992
(51) Int. Cl.: C12Q 1/04

(54) **A method for the detection of microorganisms**
Ein Verfahren zum Nachweis von Mikroorganismen
Une méthode de détection de micro-organismes

(30) Priority: 24.01.1991 FI 910358
(43) Date of publication of application: 29.07.1992
(73) Proprietor: ORION CORPORATION ORION DIAGNOSTICA, SF-02200 Espoo (FI)
(72) Inventor: Tuompo,Helena, 02150 Espoo (FI); Glasin,Heljä, 02780 Espoo (FI)
(74) Representative: VOSSIUS & PARTNER

(56) References cited:
- EP-A- 0 107 594
- EP-A- 0 288 621
- JOURNAL OF CLINICAL MICROBIOLOGY, vol. 26, no. 7, 1 July 1988, Washington, DC (US); S. ROMERO et al., pp. 1378-1382/
- METHODS OF ENZYMATIC ANALYSIS, vol. 1, 01 November 1986, Deerfield Beach, FL (US); G. MICHAL et al., pp. 197-231/

## Description

This invention relates to a method for the detection of microorganisms in liquid samples and to an apparatus using this method.

The rapid identification of certain groups or species of bacteria from biological samples can be accomplished using biochemical reactions, as in the case of enzymatic identifications and methods based on carbohydrate utilization. Particularly rapid enzymatic reactions are the oxidation-reduction reactions catalyzed by cytochromes and by enzymes such as the dehydrogenases. These reactions occur in all living cells of both animal and bacterial origin. Dehydrogenases can be assayed from living bacteria without damage to the cells by an established histochemical method, which has been used e.g. for the localization of dehydrogenases in leukocytes and in histological tissue sections (Michel, G. et al., Methods of Enzymatic Analysis, pp. 197-232, vol. 1, 1983). In this method the natural hydrogen acceptor in the dehydrogenase reaction is replaced by e.g. a tetrazolium salt, which becomes blue-violet in colour and also insoluble when reduced by hydrogen. An electron transfer mediator may also be added to the dehydrogenase reaction mixture. Such mediators, e.g. phenazine methosulphate (PMS), serve to accelerate and to amplify the reaction. PMS transfers hydrogen from NAD to the tetrazolium salt, which precipitates as the coloured compound formazan. PMS may also be replaced by other hydrogen acceptors such as menadione, meldola blue (C.I. 51175; Basic blue 6) or methoxy phenazine methosulphate.

The method described above has also been used for the identification of bacteria. When a chemical compound used in the measurement of the oxidation-reduction reaction, such as triphenyltetrazolium chloride (TTC), is added to a sample containing bacteria it is reduced and its colour changes from colourless to red. This change can be measured either visually or by colorimetric measurement. In addition to TTC other, more rapid tetrazolium salts can be used, such as iodonitrotetrazolium (INT) and neotetrazolium chloride (NTC), or alternatively salts which become blue in color, such as blue tetrazolium (BT) and nitroblue tetrazolium (NBT) (Histological and Histochemical Methods, Theory and Practice, Chapter 16, p. 258, second edition 1990).

Thus the technical problem underlying the present invention is to provide an improved method for the detection of microorganisms in liquid samples. The solution to the above technical problem is achieved by providing the embodiments characterized in the claims.

One object of the present invention is a method for the detection of microorganisms from a liquid sample such as urine or an industrial process liquid by detecting the presence of dehydrogenase enzymes of the microorganisms without disrupting the bacteria by using as a substrate for the dehydrogenase enzymes redox-compounds changing from colourless to coloured state, in which the microorganisms are separated from a suspension using a filter material (3) with a pore size small enough to prevent the passage of the microorganisms for attainment of maximum sensitivity but large enough to permit the passage of free reducing compounds.

The redox-compound is preferably dichloroindophenol, resazurin, triphenyltetrazolium chloride (TTC), iodonitrotetrazolium (INT), neotetrazolium chloride (NTC), blue tetrazolium (BT) or nitroblue tetrazolium (NBT).

A further object of the present invention is an apparatus for the detection of microorganisms in a liquid sample using the above mentioned method of the present invention, in which the microorganisms are separated from a suspension using a filter material (3) with a pore size small enough to prevent the passage of the microorganisms for attainment of optimal sensitivity but large enough to permit the passage of free reducing compounds, and the filter material in the apparatus is situated in immediate contact with an absorbent material so that the absorbent material facilitates the passage of liquid through the filter. The pore size of the filter (3) is preferably 0.75-1.2 µm.

The colour formation caused by the action of dehydrogenases, which serves to indicate the presence of bacteria in a sample, can be directly assayed from the sample without destroying the bacteria. Furthermore, Gram-negative bacteria can be distinguished from the overall bacterial population using e.g. a combination of a nonionic alkyl glucoside-type detergent and a chaotropic compound such as guanidine.

The method is performed at room temperature and is particularly suitable when the biological sample is filtered so that the bacteria remain on the filter membrane.

The method is particularly suited for use in an apparatus in which the microorganisms are assayed directly from biological samples. The apparatus comprises a porous filter or a filter composed of several units with different pore sizes, onto which the sample is transferred e.g. by suction. Microorganisms are detected immediately, on the basis of the colour change resulting from oxidation-reduction reactions, on the surface of the filter on which the bacteria were retained. Correspondingly, the amount of Gram-negative bacteria can be determined in a similar way using a suitable combination of detergents. Cells with different particle sizes, such as leukocytes, which are also reduced under the same conditions, can be separated on a different filter or using several filters and assayed individually from each filter. Free colour-reducing compounds possibly present in the sample, such as soluble enzymes, ascorbic acid, pharmaceutical compounds, low molecular weight sulfhydrylcontaining molecules, e.g. glutathione, and other reducing compounds pass through the filter on which the bacteria are trapped.

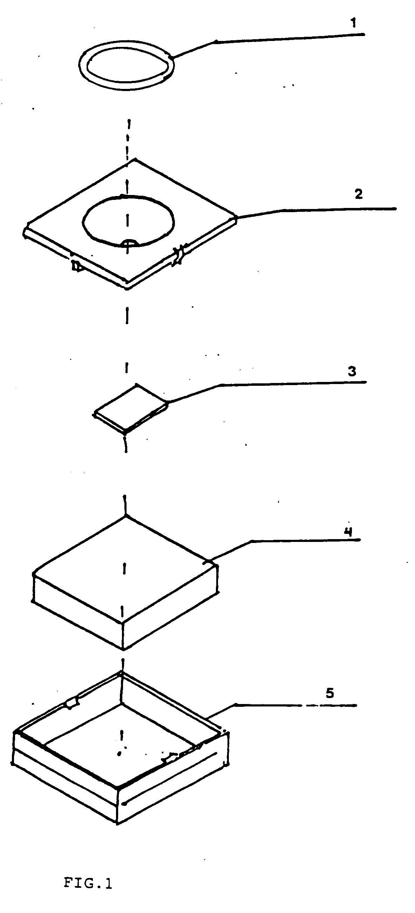

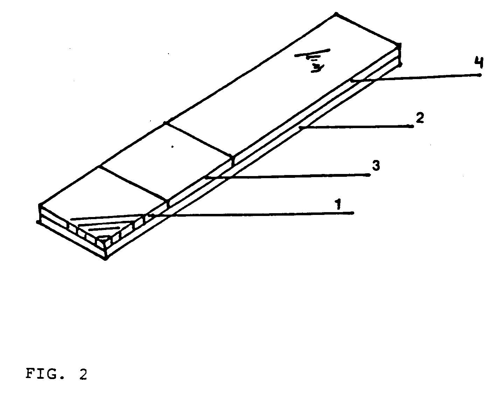

The invention is described in detail in the following with reference to the enclosed figures. Figure 1 represents the apparatus used to collect microorganisms from the sample on the filter and Figure 2 the apparatus in which the sample is sucked into the filter.

Figure 1 presents an exploded view of an apparatus in which a liquid sample is pipetted onto the prefilter 1 if the sample contains large particles or if it contains large numbers of e.g. leucocytes (in other cases prefiltration is not necessary) which may prevent visualizing the microorganismus. In the collection chamber 2 the microbes in the sample are trapped on the filter 3.

The sample may be pre-buffered.

Alternatively the microorganisms trapped on the surface of the filter 3 can be washed with a buffer solution and immediately dyed with a mixture for the detection of dehydrogenases . As a result, a blue zone due to the precipitation of formazan is formed around the microorganism. Below the filter 3 is an absorbent material 4, which absorbs the sample liquid. All the parts of the apparatus can be assembled in the base 5, in which the apparatus takes the form of a small box.

The apparatus depicted in Figure 2 includes a prefilter 1 for removing particles from the sample, a filter 3 to which microorganisms are moved from the prefilter and on which microorganisms are trapped and an absorbent material 4, all assembled on the base 2, which may be made e.g. of plastic. The filters 1 and 3 and the absorbent material 4 may be attached e.g. using two-sided tape to the base 2. The sample, buffered with a solution, is passed through the prefilter 1 and the microorganisms present in the sample are collected in the interface between it and the filter 3, from where they can be detected by passing through the prefilter 1 a mixture for the determination of dehydrogenases . As a result, blue formazan is produced around the microorganisms.

### EXAMPLE 1

The test bacteria were isolated from urinary tract infections, either ATCC type strap or clinical strains (E. coli a,b, 2956,3338, and 110515) obtained from the Department of Serology of the Universty of Helsinki. The Gram-negative bacteria were Escherichia coli (ATCC 25922) and several clinical strains: Klebsiella aerogenes (ATCC 13833), Proteus mirabilis, Pseudomonas aeruginosa and Enterobacter aerogenes (ATCC 13048). Gram-positive strains included Staphylococcus aureus (ATCC 25923), Staphylococcus saprophyticus, Staphylococcus epidermidis, Streptococcus agalactiae, Streptococcus faecium (ATCC 9790), β-hemolytic Streptococcus group B serotype, Enterococcus sp. and Corynebacterium sp. The yeast strains were Candida albicans ATCC 28367 and 36802. The bacteria were cultivated overnight in BHI broth (Brain-Heart Infusion, Difco, Detroit) at 37 °C and the bacterial count in the broth was adjusted to about 10⁸ CFU/ml (colony forming unit / ml) by dilution with a sterile 0.9 % sodium chloride solution to an optical density of 0.400 at 650nm (Perkin Elmer Spectrophotometer, Norwalk). The bacterial population was checked by dilution and plating on blood agar. The yeast strains were cultivated for 2 days at 37 °C on Sabouraud agar plates and suspensions of 10⁸ CFU/ml were made by transferring the microbial mass to sterile salt solution to an optical density of 0.400 as described above. In addition to these samples, 100 urine samples were collected. The bacterial contents of these samples were assayed by bacterial cultivation (Uricult®, Orion Diagnostica). Leucocytes possibly present in the samples were collected on a leucocyte filter (Pall Biosupport Company, Glen Cove).

In the assay 100 µl of a microorganism suspension were added to 100 µl 0.2 M Tris-HCl buffer, pH 6.5, 7.2 or 8.5 (Sigma, St. Louis) containing 1.0% glucose (BDH, Poole) or fructose (BDH, Poole), acetate (Merck, Darmstadt) or glutamate (Merck, Darmstadt). For some assays the mixture was incubated for different times on a 96-well plate. The sample was pipetted to a filtering apparatus, on which the microorganisms were collected in an area of about 3mm diameter on the surface of the filter (Schleicher and Schuell glass fiber filter No. 8), or the filter (leukocyte filter, Pall BioSupport Company, Glen Cove) was dipped into the sample and the microorganisms passed through it and transferred to the filter interface where another filter (Schleicher and Schuell No. 8) was positioned. The microorganisms were detected by adding 100 µl NBT-solution (1 mg/ml, Sigma, St. Louis) containing 10 µl PMS (1 mg/ml, Sigma, St. Louis) to the filter.

Colour formation was estimated visually after different times according to the scale:
0= colourless; 1= weak colour;
2= clearly detectable colour;
3= strongly coloured; 4= darkly coloured; 5= very darkly coloured.

### EXAMPLE 2

Detection of microorganisms from the filter at different concentrations was carried out as described in Example 1. The microorganisms were incubated for a short time on a shaker and then for 30 seconds in 0.2 M Tris-HCL buffer containing 1.0 % glucose, at different pH values. The suspension was then transferred to a filter, to which a solution of NBT-PMS was added and the blue colour due to formation of formazan was determined after 30 minutes.

| | Escherichia coli (ATCC 25922 ) | | | Escherichia coli a | | | Escherichia coli b | | | Escherichia coli 2956 | | |
|---|---|---|---|---|---|---|---|---|---|---|---|---|
| pH | 6.5 | 7.2 | 8.5 | 6.5 | 7.2 | 8.5 | 6.5 | 7.2 | 8.5 | 6.5 | 7.2 | 8.5 |
| bakt./ml | | | | | | | | | | | | |
| 10 ⁷ | 2 | 3 | 3 | 4 | 4 | 3 | 4 | 4 | 4 | 2 | 3 | 1 |
| 10 ⁶ | 2 | 2 | 2 | 2 | 2 | 2 | 0 | 2 | 3 | 1 | 1 | 1 |
| 10 ⁵ | 0 | 0 | 0 | 0 | 0 | 0 | 0 | 0 | 0 | 0 | 0 | 0 |

| | Escherichia coli 3338 | | | Escherichia coli 110515 | | | Klebsiella aerogenes (ATCC 13883) | | | Enterobacter aerogenes (ATCC 13048) | | |
|---|---|---|---|---|---|---|---|---|---|---|---|---|
| pH | 6.5 | 7.2 | 8.5 | 6.5 | 7.2 | 8.5 | 6.5 | 7.2 | 8.5 | 6.5 | 7.2 | 8.5 |
| bact./ml | | | | | | | | | | | | |
| 10 ⁷ | 4 | 5 | 5 | 2 | 3 | 3 | 5 | 5 | 5 | 5 | 5 | 5 |
| 10 ⁶ | 2 | 2 | 2 | 2 | 2 | 2 | 4 | 4 | 5 | 4 | 5 | 5 |
| 10 ⁵ | 0 | 0 | 0 | 0 | 0 | 0 | 2 | 3 | 3 | 3 | 3 | 3 |

| | Proteus mirabilis | | | Pseudomonas aerogenes | | | Enterococcus sp. | | | Streptococcus agalactiae | | |
|---|---|---|---|---|---|---|---|---|---|---|---|---|
| pH | 6.5 | 7.2 | 8.5 | 6.5 | 7.2 | 8.5 | 6.5 | 7.2 | 8.5 | 6.5 | 7.2 | 8.5 |
| bact./ml | | | | | | | | | | | | |
| 10 ⁷ | 5 | 5 | 5 | 4 | 4 | 4 | 5 | 5 | 5 | 5 | 5 | 5 |
| 10 ⁶ | 3 | 3 | 4 | 2 | 2 | 2 | 3 | 3 | 3 | 3 | 2 | 3 |
| 10 ⁵ | 1 | 1 | 2 | 1 | 2 | 2 | 2 | 2 | 2 | 1 | 2 | 1 |

| | Streptococcus beta-hemol.B | | | Streptococcus faecium (ATCC 9790 ) | | | Stafylococcus aureus | | | Stafylococcus epidermidis | | |
|---|---|---|---|---|---|---|---|---|---|---|---|---|
| pH | 6.5 | 7.2 | 8.5 | 6.5 | 7.2 | 8.5 | 6.5 | 7.2 | 8.5 | 6.5 | 7.2 | 8.5 |
| bact./ml | | | | | | | | | | | | |
| 10 ⁷ | 5 | 5 | 5 | 4 | 4 | 4 | 3 | 5 | 5 | 4 | 4 | 4 |
| 10 ⁶ | 3 | 4 | 4 | 3 | 3 | 3 | 2 | 3 | 3 | 2 | 2 | 3 |
| 10 ⁵ | 1 | 2 | 2 | 1 | 2 | 2 | 0 | 1 | 2 | 0 | 1 | 2 |

## Claims

1. A method for the detection of microorganisms comprising
(A) the separation of said microorganisms without disruption from a liquid sample using a filter material (3), and
(B) a colour-forming reaction caused by said microorganisms on the filter material (3),
wherein said reaction comprises
(a) at least one enzyme derived from said microorganisms on the filter material (3), and
(b) at least one compound capable of changing its colour upon reaction with said enzyme.

2. The method according to claim 1, wherein the reaction further comprises (c) at least one electron transfer mediator.

3. The method according to claim 2, wherein the mediator is phenazine methosulphate, menadione, meldola blue, or methoxy phenazine methosulfate.

4. The method according to any one of claims 1 to 3, wherein the liquid sample is urine or an industrial process liquid.

5. The method according to claim 1 or 4, wherein the enzyme is a dehydrogenase.

6. The method according to any one of claims 1 to 5, wherein the compound is a redox-compound.

7. The method according to claim 6, wherein the redox-compound is dichloroindophenol, resazurin, triphenyltetrazolium chloride (TTC), iodonitrotetrazolium (INT), neotetrazolium chloride (NTC) , blue tetrazolium (BT) or nitroblue tetrazolium (NBT).

8. An apparatus for the detection of microorganisms according to the method of any one of claims 1 to 7, comprising
(a) a filter material (3) which comprises at least one filtration unit having a pore size ranging from 0.75µm to 1.2µm,
(b) an absorbent material (4), wherein the filter material (3) is placed in immediate contact with the absorbent material (4) for facilitating the passage of a liquid sample through the filter material (3), and
(c) a prefitter (1).

## Patentansprüche

1. Verfahren zum Nachweis von Mikroorganismen, umfassend:
(A) die Abtrennung der Mikroorganismen ohne Aufschluß von einer flüssigen Probe unter Verwendung eines Filtermaterials (3), und
(B) eine farbgebende Reaktion, die durch die Mikroorganismen auf dem Filtermaterial (3) hervorgerufen wird, und umfaßt
(a) mindestens ein Enzym, das von den Mikroorganismen auf dem Filtermaterial (3) stammt,
(b) mindestens eine Verbindung, die nach der Reaktion mit dem Enzym die Farbe ändern kann.

2. Verfahren nach Anspruch 1, wobei die Reaktion außerdem (c) mindestens einen Elektronentransfer-Mediator umfaßt.

3. Verfahren nach Anspruch 2, wobei der Mediator Phenazinmethosulfat, Menadion, Meldola-Blau oder Methoxyphenazinmethosulfat ist.

4. Verfahren nach einem der Ansprüche 1 bis 3, wobei die flüssige Probe Urin oder eine von einem industriellen Prozess stammende Flüssigkeit ist.

5. Verfahren nach einem der Ansprüche 1 bis 4, wobei das Enzym eine Dehydrogenase ist.

6. Verfahren nach einem der Ansprüche 1 bis 5, wobei die Verbindung eine Redoxverbindung ist.

7. Verfahren nach Anspruch 6, wobei die Redoxverbindung Dichlorindophenol, Resazurin, Triphenyltetrazoliumchlorid (TTC), Iodonitrotetrazolium (INT), Neotetrazoliumchlorid (NTC) , Blautetrazolium (BT) oder Nitroblautetrazolium (NBT) ist.

8. Apparatur zum Nachweis von Mikroorganismen gemäß dem Verfahren nach einem de Ansprüche 1 bis 7, umfassend
(a) ein Filtermaterial (3), das mindestens eine Filtrationseinheit mit einer Porengröße zwischen 0.75 µm und 1.2 µm umfaßt,
(b) ein absorbierendes Material (4), wobei das Filtermaterial (3) in direkten Kontakt mit dem absorbierenden Material (4) gebracht wird, um den Durchlauf einer flüssigen Probe durch das Filtermaterial (3) zu erleichtern, und
(c) ein Vorfilter (1).

## Revendications

1. Procédé pour la détection de micro-organismes comprenant
(A) la séparation desdits micro-organismes sans lyse, à partir d'un échantillon liquide, au moyen d'un matériau filtrant (3), et
(B) une réaction de coloration provoquée par lesdits micro-organismes sur le matériau filtrant (3),
ladite réaction comprenant
(a) au moins une enzyme provenant desdits micro-organismes présents sur le matériau filtrant (3), et
(b) au moins un composé capable de changer de couleur lors de la réaction avec ladite enzyme.

2. Procédé selon la revendication 1, dans lequel la réaction comprend (c) au moins un médiateur de transfert d'électrons.

3. Procédé selon la revendication 2, dans lequel le médiateur est le méthosulfate de phénazine, la ménadione, le bleu Meldola ou le méthosulfate de méthoxyphénazine.

4. Procédé selon l'une quelconque des revendications 1 à 3, dans lequel l'échantillon liquide est de l'urine ou un liquide de processus industriel.

5. Procédé selon la revendication ou 4, dans lequel l'enzyme est une déshydrogénase.

6. Procédé selon l'une quelconque des revendications 1 à 5, dans lequel le composé est un composé rédox.

7. Procédé selon la revendication 6, dans lequel le composé rédox est le dichloro-indophénol, la résazurine, le chlorure de triphényltétrazolium (CTT), l'iodonitrotétrazolium (INT), le chlorure de néotétrazolium (NTC), le bleu de tétrazolium (BT) ou le bleu nitré de tétrazolium (BNT).

8. Appareil pour la détection de micro-organismes selon le procédé de l'une quelconque des revendications 1 à 7, comprenant
(a) un matériau filtrant (3) comprenant au moins une unité de filtration ayant une taille de pores allant de 0,75 µm à 1,2 µm,
(b) un matériau absorbant (4), le matériau filtrant (3) étant mis en contact immédiat avec le matériau absorbant (4) pour faciliter le passage d'un échantillon liquide à travers le matériau filtrant (3), et
(c) un préfiltre.
